(19) Europäisches Patentamt
European Patent Office
Office européen des brevets

(11) Numéro de publication: **0 424 293 B1**

(12) **FASCICULE DE BREVET EUROPEEN**

(45) Date de publication de fascicule du brevet: **12.04.95**

(51) Int. Cl.[6]: **C12Q 1/04**, C12Q 1/18, C07D 265/38

(21) Numéro de dépôt: **90420453.4**

(22) Date de dépôt: **18.10.90**

Le dossier contient des informations techniques présentées postérieurement au dépôt de la demande et ne figurant pas dans le présent fascicule.

# (54) Procédé pour la détection de microorganismes impliquant l'utilisation de résorufine ou d'orcirufine.

(30) Priorité: **20.10.89 FR 8914087**

(43) Date de publication de la demande:
**24.04.91 Bulletin 91/17**

(45) Mention de la délivrance du brevet:
**12.04.95 Bulletin 95/15**

(84) Etats contractants désignés:
**AT BE CH DE DK ES FR GB GR IT LI LU NL SE**

(56) Documents cités:
**EP-A- 0 286 434**

**JOURNAL CHEM. TECHN. BIOTECHNOLOGY, vol. 34B, 1984; S.D. ROLLER et al., pp. 3-12&NUM;**

**BIOCHEMICAL PHARMACOLOGY, vol. 32, no. 1, 1983; pp. 175-176&NUM;**

**BIOCHEMICAL & BIOPHYSICAL RESEARCH COMMUNICATIONS, vol. 136, no. 2, 1986; pp. 555-562&NUM;**

**CYTOMETRY, vol. 9, 1988; pp. 394-404&NUM;**

**NATURE, vol. 155, 1945; pp. 401-402&NUM;**

**ZBL. BAKT. HYG., 1. Abt. Orig. B19, 1984; pp. 217-224&NUM;**

(73) Titulaire: **BIO MERIEUX Société anonyme dite:**
**Marcy l'Etoile**
**F-69260 Charbonnieres les Bains (FR)**

(72) Inventeur: **Monget, Daniel**
**24 Résidence du Moulin**
**F-01150 Saint Sorlin en Bugey (FR)**

(74) Mandataire: **Guerre, Dominique et al**
**Cabinet Germain et Maureau**
**20 Boulevard Eugène Deruelle**
**BP 3011**
**F-69392 Lyon Cédex 03 (FR)**

EP 0 424 293 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de manière générale la détection de micro-organismes, dans différents milieux ou sur différents substrats, pour différentes fins ou applications, telles que l'identification ou la détection microbienne.

Plus précisément, l'invention s'intéresse aux réactifs, comprenant un milieu réactionnel en phase aqueuse, lequel contient au moins une source de carbone, ainsi qu'un marqueur luminescent dont une émission lumineuse est modifiable en conséquence du développement ou du métabolisme du micro-organisme à détecter, introduit dans le milieu réactionnel.

Par micro-organisme, on entend notamment les microbes, bactéries, et levures.

Par "émission lumineuse", on entend tout rayonnement lumineux émis sous l'effet d'une excitation extérieure, notamment lumineuse, dans le domaine du visible ou non, susceptible d'être modifiée par le développement du micro-organisme dans le milieu réactionnel, et d'être observée, contrôlée ou mesurée par tous moyens de détection optique, dont la simple observation à l'oeil nu. Ressortent de cette définition, différentes propriétés lumineuses, telles que la luminescence, la fluorescence, la phosphorescence, visibles ou non à l'oeil nu par exemple.

Depuis de nombreuses années, les fabricants et professionnels des réactifs ou méthodes de détection de micro-organismes sont à la recherche d'une solution présentant ensemble les caractéristiques essentielles suivantes :

- avoir un caractère universel, c'est-à-dire indépendante du type ou de l'espèce des micro-organismes recherchés
- être substantiellement indépendante de la nature du milieu ou du substrat dans lequel les micro-organismes sont recherchés
- présenter une grande sensibilité.

Aucune des méthodes disponibles à ce jour, y compris les plus récentes, ne permet de répondre à toutes ces exigences, comme montré et détaillé ci-après.

Une première méthode, et c'est la plus simple, consiste à prélever un échantillon, à éventuellement diluer ce dernier de manière successive, et à appliquer l'échantillon obtenu à la surface d'un milieu nutritif gélosé. Après incubation pendant 24 à 48 heures, on procède par des moyens manuels ou vidéo, au contrôle de la présence des micro-organismes, et éventuellement à leur comptage. La technique consistant à immerger directement dans le milieu contrôlé des lames imprégnées avec un milieu nutritif, et à contrôler, après incubation, la présence de micro-organismes sur ces lames, constitue en fait une variante de cette première méthode.

Une telle solution est peu sensible, et peu fiable, en particulier lorsque des moyens de contrôle vidéo sont utilisés. Une telle méthode nécessite également d'adapter ou choisir le milieu nutritif, en fonction des micro-organismes recherchés.

Une deuxième méthode, assez proche de la première, consiste à pigmenter ou colorer l'échantillon prélevé, ou provoquer sa fluorescence, avec toutes substances appropriées telle que l'acridine orange, puis à observer directement l'échantillon ainsi traité, par exemple au microscope. Pour l'essentiel, une telle méthode présente des inconvénients comparables à ceux de la première méthode ; en particulier, une telle solution demeure manuelle, et son automatisation apparaît difficile.

Une troisième méthode consiste à prélever un échantillon, et à mettre en culture ce dernier pendant un temps prédéterminé, puis à observer le milieu de culture par néphélométrie ou turbidimétrie. En d'autres termes, la présence de micro-organismes est révélée par l'opacité du milieu de culture. Une telle méthode apparaît assez peu sensible, et l'opacité observée ne résulte pas uniquement de la présence des micro-organismes, de telle sorte que cette solution n'apparaît pas utilisable ou exploitable pour des milieux biologiques complexes, par exemple des échantillons de sang.

Différentes méthodes plus élaborées, par voie biochimique, et faisant appel à la colorimétrie ou la fluorimétrie, ont été proposées pour détecter des micro-organismes.

Une quatrième méthode consiste à mettre en présence un échantillon à contrôler et un réactif comprenant un hydrate de carbone comme le glucose, et un indicateur de pH. Comme le développement, ou le métabolisme du micro-organisme génère des acides, ceux-ci modifient la coloration de l'indicateur de pH. Une telle solution s'avère assez peu sensible, et n'a pas en tout cas un caractère universel.

Une cinquième méthode consiste à mettre en présence un échantillon à contrôler, et un indicateur chromogène de réduction ou d'oxydo-réduction. Aussi, conformément à la publication "Zbl. Bakt. Hyg. 1984, pages 217-224", on a proposé d'utiliser la résazurine, en tant qu'indicateur coloré (bleu) de réduction. Les mécanismes biochimiques de la chaîne respiratoire des micro-organismes présents génèrent des substances réductrices telle que NADH (Nicotinamide-Adénine-Dinucleotide Hydrogéné) ; cette substance

réductrice conduit à la réduction de l'indicateur, en l'espèce sa transformation en résorufine, et donc à sa modification de couleur, en l'espèce au passage d'une couleur bleue à une couleur rose. Pour ce faire, on met en oeuvre le procédé de détection suivant :

(a) on dispose de ou on obtient le milieu réactionnel en phase aqueuse, contenant au moins une source de carbone, et le marqueur, en l'occurrence la résazurine, dont la couleur bleue est susceptible d'être modifiée en rose par le développement du micro-organisme dans le milieu réactionnel

(b) on met en présence le milieu réactionnel avec l'échantillon, puis on incube ledit milieu

(c) après ou lors de l'incubation, on observe le changement de couleur dudit milieu.

Une telle méthode est assez peu sensible, et ne permet de détecter qu'un nombre limité de micro-organismes.

Une sixième méthode consiste à utiliser des substrats enzymatiques de synthèse, chromogéniques ou fluorogéniques. La présence de certaines enzymes, liées au développement des micro-organismes, conduit à couper le substrat enzymatique en deux, et à libérer la partie chromogène ou fluorogène, laquelle peut être alors détectée.

Une telle méthode ne présente pas de caractère universel, en ce sens que le substrat enzymatique doit être à chaque fois adapté au type de micro-organisme recherché ; en particulier, il n'existe pas une seule et même enzyme commune à tous les micro-organismes. Par ailleurs, pour des milieux biologiques complexes, tels que le sang ou l'urine, contenant déjà des enzymes, cette méthode ne permet pas de distinguer ceux spécifiques aux micro-organismes, par exemple bactéries ou microbes, donc on recherche la présence.

Une septième méthode consiste à détecter les micro-organismes, par l'intermédiaire du métabolite ATP (Adénosine-Tri-Phosphate), en présence d'un substrat luciférine, et par oxydation de ce dernier en présence de luciférase, laquelle conduit à la bioluminescence du substrat précité. Une telle méthode présente pour l'essentiel les mêmes inconvénients que ceux énoncés pour la méthode précédente.

Une huitième méthode procède par la mise en évidence du gaz carbonique produit par les micro-organismes, en présence d'une ou plusieurs sources de carbone dans le milieu réactionnel ; une telle technique est par exemple utilisée en hémoculture pour déceler une présence microbienne. La détection du gaz carbonique peut être radiométrique, auquel cas on doit utiliser une source de carbone marquée au C 14, ou spectro-photométrique dans l'infra-rouge. On peut aussi mesurer la production de gaz carbonique par le contrôle de l'augmentation de pression dans un système fermé, à l'aide d'un manomètre ou d'un capteur de pression approprié. Une telle méthode est assez peu sensible, et dans le cas d'un marqueur radio-actif, elle pose différents problèmes de sécurité et agrément administratif préalable du laboratoire de mesure.

Une dernière méthode consiste à détecter les micro-organismes, par l'intermédiaire de l'évolution des paramètres électriques du milieu réactionnel dans lequel les micro-organismes se développent ; ces paramètres étant notamment l' impédance, la conductance ou la capitance. Sur la base de cette méthode, différents bio-capteurs ont été développés et mis sur le marché, notamment dans le domaine agro-alimentaire. Une telle technique est assez peu sensible, en particulier vis-à-vis des exigences de diagnostic à des fins médicales. Et les matériels disponibles actuellement selon cette solution sont assez peu performants.

Au terme de cette analyse des solutions antérieures en matière de détection de micro-organisme, on observera au passage que certaines de ces méthodes procèdent par échantillonnage, c'est-à-dire le prélèvement d'un échantillon sur le milieu ou sur le substrat à contrôler, la culture de cet échantillon, et éventuellement le prélèvement à intervalles réguliers du milieu de culture. De telles opérations, en général effectuées de manière manuelle, paraissent difficilement automatisables.

La présente invention a pour objet un nouveau mode d'identification ou caractérisation bactériologique, mettant en oeuvre un réactif d'oxydo-réduction présentant une émission lumineuse pouvant être détectée ou mesurée pour l'identification du ou des micro-organismes. L'invention a recherché un mode présentant, d'une part un caractère universel, c'est-à-dire substantiellement indépendant et du milieu dans lequel on recherche les micro-organismes, et de la nature ou espèce de ces derniers, et d'autre part une bonne sensibilité, autorisant une automatisation et assurant une détection des micro-organismes dans une concentration relativement faible.

Grâce à la découverte expérimentale exposée ci-après. on a trouvé un réactif répondant à l'ensemble des exigences précédentes.

Cette procédure expérimentale a été la suivante :

Sous l'action de la $\beta$-glucuronidase d'Escherichia coli, cultivée dans un bouillon approprié, il est possible de couper le substrat 4-méthylumbelliferyl-$\beta$D glucuronide, de libérer ainsi la 4-méthylumbelliferone fluorescente, et donc détecter la bactérie précitée.

**1ère expérience**

En remplaçant le substrat 4-méthylumbelliferyl-$\beta$D-glucuronide par le substrat résorufine-$\beta$D-glucuronide pour la recherche de la $\beta$-glucuronidase d'E. coli, dans le même bouillon, on a constaté que bien que le nouveau substrat soit hydrolysé au cours de la croissance bactérienne en ampoule, la fluorescence rose de la résorufine ne s'exprime pas ; on obtient tout au plus un liseret rose à l'interface bouillon/atmosphère gazeuse, alors que la résorufine est soluble en milieux aqueux.

**2ème expérience**

Dans des ampoules stériles contenant le même bouillon que précédemment, on introduit de la résorufine libre à la concentration de 20 micromoles/litre. Les ampoules sont ensuite ensemencées par différentes souches bactériennes d'entérobactéries, à raison de $10^6$ bactéries/ml.

Au départ, le bouillon est rose dans toutes les ampoules (fluorescence de la résorufine).

Au-delà de 4 heures d'incubation à 37° C, le milieu des ampoules montrant une croissance est décoloré (avec parfois un liseret rose en surface), alors que le témoin sans bactérie reste rose.

Il résulte de ces deux expériences que la croissance bactérienne en bouillon en présence de résorufine entraîne la disparition de la fluorescence de marqueur, et donc que la résorufine apparaît être un excellent indicateur de la croissance bactérienne.

Conformément à la publication "Nature, Vol 155, 1945, pages 401-402", on savait depuis très longtemps que la résorufine pouvait être transformée de manière réversible en dihydrorésorufine, à la fois par réduction et en milieu acide. Mais, jusqu'à la découverte énoncée précédemment, personne n'avait songé à utiliser cette réaction d'oxydo-réduction pour une caractérisation bactériologique.

A partir de cette découverte, la présente invention concerne l'utilisation de résorufine ou orcirufine, ou leur forme anionique, pour l'obtention d'un réactif de détection de micro-organismes. L'invention concerne aussi un procédé de détection de micro-organismes dans un échantillon, tel que décrit par référence à la publication "Zbl. Bakt. Hyg. 1984, pages 217-224", selon lequel le marqueur fluorescent est la résorufine ou l'orcirufine, ou leur forme anionique, et lors de l'étape (c), c'est-à-dire pour l'observation de l'émission lumineuse du milieu, on mesure la fluorescence du marqueur, dont la diminution ou l'extinction traduit la présence d'au moins un micro-organisme dans l'échantillon.

La résorufine et l'orcirufine répondent aux deux formules exprimées respectivement ci-après.

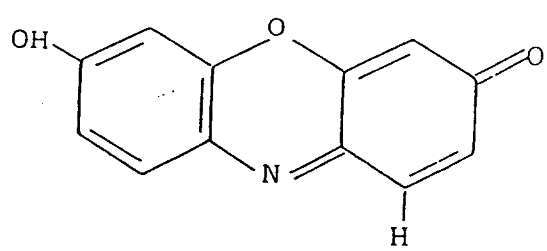

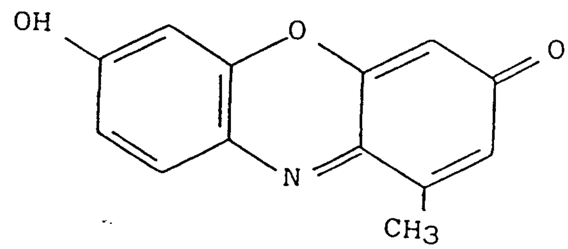

Comme la fluorescence des marqueurs précités a pour origine une délocalisation électronique importante entre les positions 3 et 7, générant un hybride de résonnance selon la formule suivante :

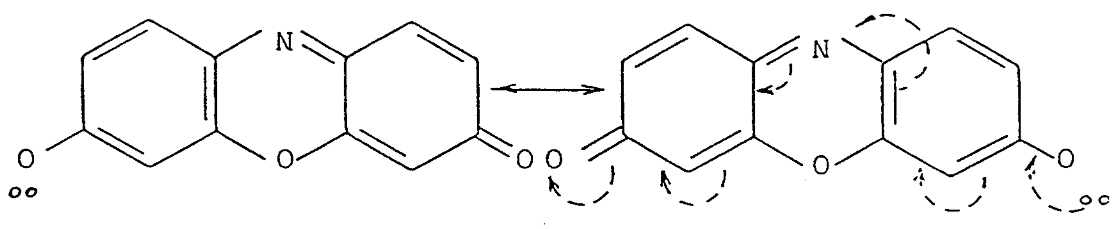

la présente invention s'est intéressée à titre de marqueur, aux structures chimiquement proches, répondant aux deux formules générales ci-après, et présentant également des propriétés de fluorescence utilisables dans le cadre d'un réactif selon l'invention, pour la détection de micro-organismes.

Il s'agit de composés chimiques, sous forme neutre ou anionique, répondant à la formule suivante :

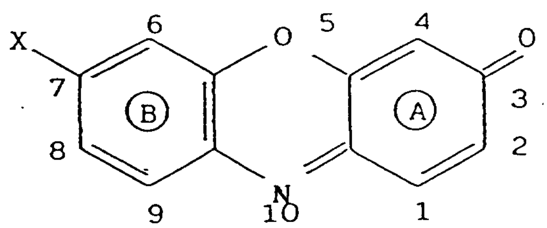

dans laquelle :

- les positions 6, 8, 9 sont occupées chacune par un atome d'hydrogène ou un substituant choisi dans le groupe comprenant le fluor, le chlore, le brome, des substitants alkyle, alkoxy, carboxylique, amide et cyano,
- les positions 1 et 4 sont occupées chacune par un atome d'hydrogène ou un substituant choisi dans le groupe comprenant le fluor, le chlore, le brome, des substituants alkyle, alkoxy, carboxylate, carboxylique, amide et cyano, et la position 2 est occupée par un atome d'hydrogène, ou un substituant choisi dans le groupe comprenant le chlore, le brome et un substituant carboxylique ; ou les positions 1 et 2 appartiennent en commun au noyau A et à un noyau insaturé,
- X est une fonction hydroxyle.

Des composés répondant à cette dernière formule générale, ainsi que leur mode d'obtention sont précisés dans le tableau ci-après.

Selon la présente invention, les conditions suivantes sont mises en oeuvre:

- le pH du milieu réactionnel est compris entre 6,0 et 8,0

- l'indicateur ou marqueur est contenu dans le milieu réactionnel,

| Substituant / nom du composé | X | 1 | 2 | 4 | 6 | 8 | 9 | OBTENTION |
|---|---|---|---|---|---|---|---|---|
| Résorufine | OH | H | H | H | H | H | H | Condensation quinone-monochloroimine (2) avec résorcinol ; ou réduction puis oxydation à l'air de résazurine (3) ; ou chauffage résorcinol, nitrobenzène et acide sulfurique, neutralisation puis extraction à l'éthanol |
| Orcirufine | OH | $CH_3$ | H | H | H | H | H | Condensation quinone-monochloroimine (2) avec orcinol |
| Résorufamine | $NH_2$ | H | H | H | H | H | H | Condensation N,N'-dichloroquinone diimine (1) et résorcinol |
| Orcirufamine | $NH_2$ | $CH_3$ | H | H | H | H | H | Condensation N,N'-dichloroquinone diimine (1) et orcinol |
| Méthylorcirufine | OH | $CH_3$ | H | H | H | H | $CH_3$ | Réaction en solution éther d'orcinol avec l'acide nitrique fumant, pour obtenir dimethyl-resazurine ; réduction puis oxydation à l'air de la dimethyl-resazurine |
| Pentylrésorufine | OH | $C_5H_{11}$ | H | H | H | H | H | idem méthylorcirufine, à partir d'olivetol (5-pentylresorcinol) |
| 2-Chlororésorufine | OH | H | Cl | H | H | H | H | idem méthylorcirufine, mais à partir du 4-chlororésorcinol |
| 6,8-Dichloro-1-méthyl-résorufine | OH | $CH_3$ | H | H | Cl | Cl | H | Condensation réactif (4) de Gibbs avec orcinol |

| nom du composé \ substituant | X | 1 | 2 | 4 | 6 | 8 | 9 | OBTENTION |
|---|---|---|---|---|---|---|---|---|
| 6,8-Dibromo-1-Méthyl résorufine | OH | $CH_3$ | H | H | Br | Br | H | Condensation réactif (4) de Gibbs avec orcinol |
| 3-Méthoxy-Résorufine | OH | $OCH_3$ | H | H | H | H | H | idem méthylorcirufine, mais à partir du 5-méthoxy-résorcinol |
| 2-Carboxyl-Résorufine | OH | H | $CO_2H$ | H | H | H | H | Condensation quinone-monochloroimine (2) avec acide β-résorcinique (acide 2-4-dihydroxybenzoïque) |
| Naphto-Résorufine | OH | 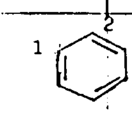 | | H | H | H | H | Condensation N-chloroquinone monoimine (2) avec naphto-résorcinol |
| 4-Méthyl-résorufine | OH | H | H | $CH_3$ | H | H | H | Condensation |
| 2,4,6,8-tétrabromo-résorufine | OH | H | Br | Br | Br | Br | H | Halogénation directe de la résorufine (ou orcirufine) |
| 2,4,6,8-Tétrachloro-résorufine | OH | H | Cl | Cl | Cl | Cl | H | Halogénation directe de la résorufine (ou orcirufine) |

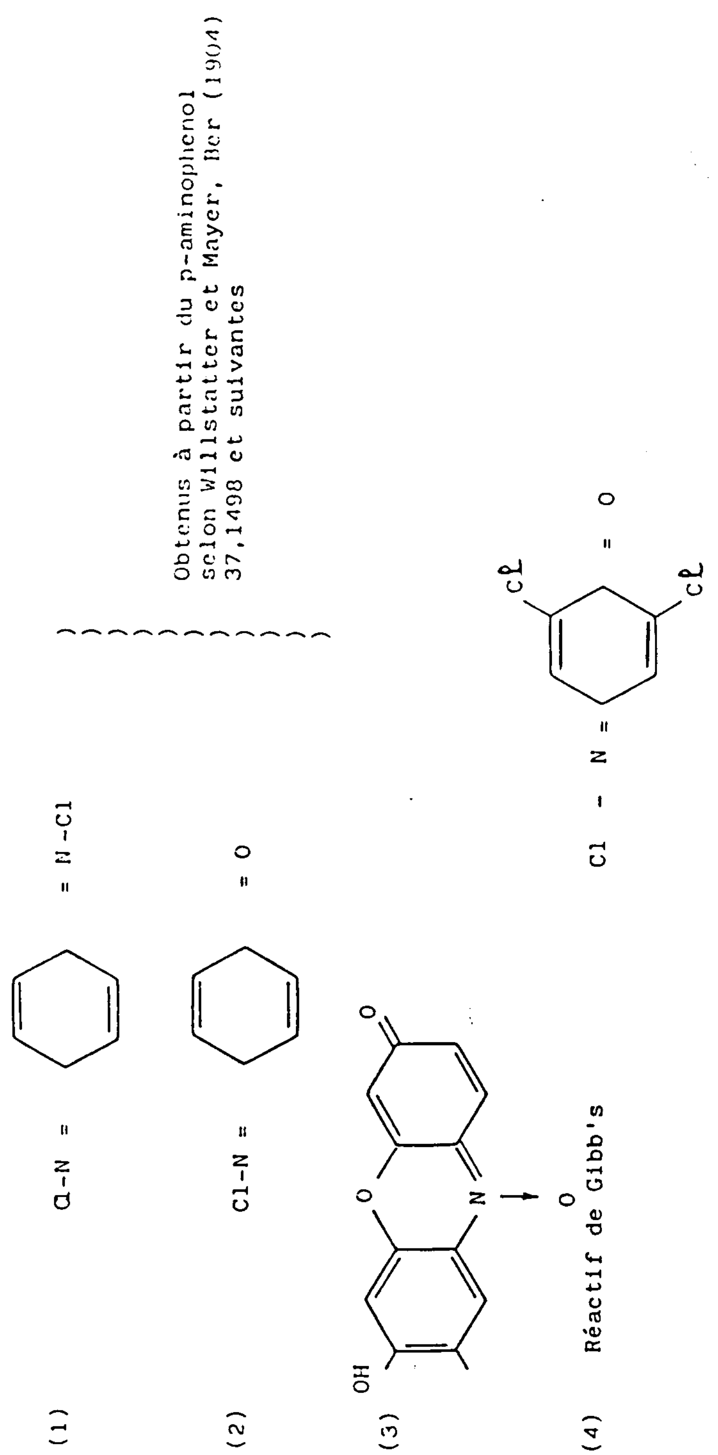

(1)

(2)

Obtenus à partir du p-aminophenol selon Willstatter et Mayer, Ber (1904) 37,1498 et suivantes

(3)

(4) Réactif de Gibb's

à raison de 0,01 mg/l à 100 mg/l, et de préférence de 1 à 10 mg/l

- la source d'azote du milieu réactionnel peut être de toute nature, par exemple peptone ou sulfate d'ammonium
- la source de carbone peut être de tous types, telle que glucose, lactose, ou autres

S'agissant du milieu réactionnel, contenant les sources d'azote et de carbone nécessaires aux reactifs, la majorité des bouillons commercialisés peuvent convenir, par exemple trypticase-soja, coeur-cervelle, etc...

Un procédé de détection conforme à l'invention consiste à mettre en oeuvre les étapes suivantes :

1) Le réactif, comprenant comme précédemment le milieu réactionnel et le marqueur selon l'invention est préparé, réparti en flacons ou ampoules, ou toutes autres formes de conditionnement, et stérilisé par

filtration ou autoclave.

2) Le réactif est mélangé à l'échantillon, milieu, ou substrat à contrôler.

3) Le mélange obtenu est incubé entre 20 ° C et 65 ° C, pendant un temps compris entre 1 minute et 7 jours, par exemple.

4) la fluorescence du marqueur est mesurée à intervalles réguliers, à l'aide d'un fluorimètre, ou simplement appréciée à l'oeil nu.

La diminution de la fluorescence, voire son extinction totale, traduit la présence de micro-organismes dans l'échantillon ou substrat contrôlé.

La présente invention est maintenant décrite et détaillée, quant aux expériences effectuées, par rapport à la résorufine. Ce marqueur, apporte en outre les avantages supplémentaires et importants suivants :

- ce produit n'est pas toxique
- il présente une grande sensibilité, autorisant son emploi à des concentrations très faibles, de l'ordre de 1 à 2 mg/l
- et surtout, s'agissant de la fluorescence, les longueurs d'ondes maximum d'excitation et d'émission se situent respectivement à 572 nm et 585 nm ; ces longueurs d'ondes se situent au-delà des radiations absorbées par la plupart des milieux ou échantillons analysés, tout en restant dans le domaine du visible, ce qui permet d'observer de manière séparée la fluorescence spécifique à la résorufine, sans interférence ou gêne du milieu ambiant.

## Exemple 1 : Préparation du réactif

Le milieu réactionnel est préparé en mélangeant les ingrédients suivants :

| | |
|---|---|
| - bouillon Mueller Hinton déshydraté (Difco, réf 0757-01) | 21 g |
| - glucose (Merck, réf 8337) | 5 g |
| - résorufine (Aldrich, réf 23015-4) | 2 mg |
| - eau distillée | 1000 ml |
| - pH | 7,3 |

Ce milieu est réparti en tubes de verre avec bouchons à vis, à raison de 6 millilitres par tube, et autoclavé à 121 ° C pendant 15 minutes.

Les tubes stérilisés sont conservés à l'obscurité, la résorufine étant sensible à la lumière.

Ce milieu sera utilisé dans les exemples d'applications 2 à 4 selon la méthode générale suivante : après avoir ajouté le ou les différents éléments spécifiques à l'application considérée, les tubes vissés sont agités dans une étuve sur un agitateur Vibratest réglé à 100 tours/minute. La mesure de la fluorescence est effectuée à l'aide d'un fluorimètre LKB type LS - 5B. Les tubes sont introduits directement dans le porte-cuves. La longueur d'ondes d'excitation est réglée à 572 nm et celle d'émission à 585 nm. Il est à noter que dans l'exemple 4, le glucose présent dans le milieu réactionnel peut être remplacé par d'autres sucres.

## Exemple 2 : Application à l'hémoculture

Dans 7 tubes de verre contenant le réactif préparé selon l'exemple 1, il est ajouté du sang frais humain stérile, à raison de 1 millilitre par tube.

Le premier tube est utilisé comme témoin et les 6 autres sont ensemencés par l'une des 6 souches microbiennes suivantes :

- Ec = Escherichia coli
- Cf = Citrobacter freundii
- Pa = Pseudomonas aeruginosa
- Sa = Staphylococcus aureus
- Ef = Enteroccocus faecalis
- Ca = Candida albicans

La quantité de micro-organismes introduite au départ dans chaque tube est de 100 cellules viables par millilitre de milieu. Cet inoculum est contrôlé par comptage des colonies formées à la surface d'une gélose au sang après 24 heures d'incubation.

La mesure de la fluorescence est effectuée après 12 h, 24 h, 36 h et 48 h d'incubation à 35 ° C. L'intensité de la fluorescence est reportée dans le tableau ci-dessous. L'extinction partielle ou totale de la fluorescence témoigne de la présence de cellules microbiennes dans les échantillons de départ

| Souche / Incubat. | témoin négatif | Ec | Cf | Ps | Sa | Ef | Ca |
|---|---|---|---|---|---|---|---|
| 12 H | 463 | 11 | 454 | 465 | 447 | 459 | 461 |
| 24 H | 465 | 9 | 10 | 464 | 460 | 126 | 460 |
| 36 H | 458 | 8 | 10 | 53 | 27 | 13 | 461 |
| 48 H | 464 | 9 | 9 | 17 | 13 | 18 | 19 |

Pour cette application, il est préférable d'utiliser un bouillon spécifique à l'hémoculture, à la place du bouillon Mueller-Hinton.

**Exemple 3 : Application à l'antibiogramme**

6 antibiotiques différents sont introduits dans des tubes préparés comme indiqué dans l'exemple 1. Chaque antibiotique est utilisé aux 2 concentrations "c" et "C" indiquées dans le tableau ci-après. Des tubes sans antibiotique sont utilisés comme témoins.

Chaque couple de tubes correspondant à un antibiotique aux 2 concentrations "c" et "C" est ensemencé par l'une des 3 souches microbiennes suivantes, à raison de 10 cellules viables par millilitre :

- Ec = Escherichia coli ATCC 25922
- Pa = Pseudomonas aeruginosa ATCC 27853
- Sa = Staphylocossus aureus ATCC 25923

La mesure de la fluorescence est effectuée après 18 heures d' incubation à 35° C. L'intensité de la fluorescence est reportée dans le tableau ci-dessous.

Les résultats sont symbolisés selon :

- S = souche sensible = fluorescence maximale
- R = souche résistante = extinction totale de la fluorescence
- I = souche intermédiaire = extinction partielle de la fluorescence

L'antibiogramme est contrôlé par la technique de la dilution en gélose.

| Antibiotique | Concentration en µg / ml | Ec | | Pa | | Sa | |
|---|---|---|---|---|---|---|---|
| Témoin | c = 0 | 8 | | 10 | | 9 | |
| | C = 0 | 7 | | 11 | | [illegible] | |
| Pénicilline | c = 0,25 | 9 | R | 10 | R | 455 | S |
| | C = 16 | 8 | | 9 | | 457 | |
| Pipéracilline | c = 32 | 460 | S | 463 | S | 459 | S |
| | C = 128 | 465 | | 450 | | 468 | |
| Céfoxitine | c = 8 | 439 | S | 15 | R | 448 | S |
| | C = 32 | 463 | | 9 | | 457 | |
| Rénamycine | c = 2 | 15 | I | 13 | R | 438 | S |
| | C = 16 | 458 | | 10 | | 447 | |
| Trétracycline | c = 1 | 451 | S | 15 | R | 443 | S |
| | C = 4 | 465 | | 19 | | 459 | |
| Erythromycine | c = 1 | 8 | R | 11 | R | 445 | S |
| | C = 4 | 10 | | 13 | | 460 | |

L'antibiogramme selon l'invention peut être réalisé en cupules miniaturisées (plaque de microtitration).

**Exemple 4 : Application**

L'utilisation de 7 différents sucres par 8 souches microbiennes à des fins d'identification est présentée dans cet exemple :

Les 7 sucres testés sont :

- Glucose (GLU)
- Maltose (MAL)
- Saccharose (SAC)
- Lactose (LAC)
- Cellobiose (CEL)
- Tréhalose (TRE)
- Mélibiose (MEL)

Les tests sont pratiqués dans le réactif en tubes de verre, préparé comme indiqué dans l'exemple 1. Le glucose contenu dans ce milieu est remplacé le cas échéant par le sucre correspondant, à la même concentration de 5 g/l.

La batterie de tubes est ensemencée par les 8 souches suivantes :

- Ca = Candida albicans
- Ct = Candida tropicalis
- Tg = Torulopsis glabrata

- Ec = Escherichia coli
- Kp = Klebsiella pneumoniae
- Pv = Proteus vulgaris
- Ah = Aeromonas hydrophila
- Va = Vibrio alginolyticus

Pour chaque souche, une suspension est réalisée en sérum physiologique, ajustée au point 2 de l'échelle de McFarland. 200 microlitres de cette suspension sont introduits dans les 7 tubes contenant chacun des sucres.

La mesure de la fluorescence est effectuée après 18 heures d'incubation à 35° C. L'intensité de la fluorescence est reportée dans le tableau ci-dessous. Les résultats sont symbolisés en :

- réaction négative... (-) = fluorescence maximale
- réaction positive... (+) = extinction de la fluorescence

Les résultats obtenus sont contrôlés à l'aide de microméthodes commercialisées avec les tests commercialisés sous la marque API par la société BIO MERIEUX, sous les noms : API 20 E, API 20 C, API 5O CH.

| Sucre / Souche | GLU | MAL | SAC | LAC | CEL | TRE | MEL |
|---|---|---|---|---|---|---|---|
| Ca | 31<br>+ | 25<br>+ | 37<br>+ | 466<br>− | 453<br>− | 19<br>+ | 439<br>− |
| Ct | 12<br>+ | 17<br>+ | 21<br>+ | 441<br>− | 16<br>+ | 20<br>+ | 452<br>− |
| Tg | 12<br>+ | 456<br>− | 447<br>− | 452<br>− | 449<br>− | 16<br>+ | 465<br>− |
| Ec | 9<br>+ | 13<br>+ | 459<br>− | 10<br>+ | 454<br>− | 10<br>+ | 11<br>+ |
| Kp | 10<br>+ | 9<br>+ | 9<br>+ | 11<br>+ | 8<br>+ | 10<br>+ | 8<br>+ |
| Pv | 9<br>+ | 469<br>+ | 10<br>+ | 458<br>− | 461<br>− | 437<br>− | 456<br>− |
| Ah | 10<br>+ | 14<br>− | 11<br>− | 451<br>− | 467<br>− | 12<br>+ | 444<br>− |
| Va | 11<br>+ | 10<br>+ | 8<br>+ | 469<br>− | 451<br>− | 10<br>+ | 449<br>− |

L'utilisation de sources de carbone à des fins d'identification selon l'invention peut être faite en cupules miniaturisées (plaques de microtitration par exemple).

Par conséquent, les réactifs selon l'invention peuvent être utilisés pour la détection de micro-organismes dans toutes sortes d'échantillons biologiques, tels que fluides corporels (sang, urine, liquide céphalo-rachidien, etc...), produits alimentaires, eau, produits pharmaceutiques, cosmétiques, etc...

De manière similaire à l'antibiogramme selon l'exemple 3, l'invention peut être utilisée pour effectuer un antifongigramme, auquel cas on utilise une pluralité de réactifs selon l'invention, différant les uns des autres uniquement par l'ajout d'anti-fongiques respectivement différents.

## Revendications

1. Utilisation d'un composé chimique répondant à la formule suivante, ou sa forme anionique,

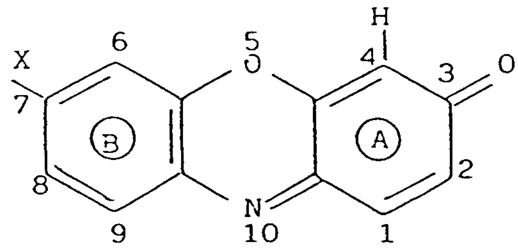

dans laquelle :

- les positions 6, 8, 9 sont occupées chacune par un atome d'hydrogène ou un substituant choisi dans le groupe comprenant le fluor, le chlore, le brome, des substitants alkyle, alkoxy, carboxylique, amide et cyano,
- les positions 1 et 4 sont occupées chacune par un atome d'hydrogène ou un substituant choisi dans le groupe comprenant le fluor, le chlore, le brome, des substituants alkyle, alkoxy, carboxylate, carboxylique, amide et cyano, et la position 2 est occupée par un atome d'hydrogène, ou un substituant choisi dans le groupe comprenant le chlore, le brome et un substituant carboxylique; ou les positions 1 et 2 appartiennent en commun au noyau A et à un noyau insaturé,
- X est une fonction hydroxyle, pour l'obtention d'un réactif de détection de micro-organismes.

2. Utilisation selon la revendication 1, caractérisé en ce que le composé chimique est l'orcirufine ou la résorufine.

3. Procédé de détection de micro-organismes dans un échantillon, selon lequel :
(a) on dispose de ou on obtient un milieu réactionnel en phase aqueuse, contenant au moins une source de carbone, et un marqueur dont l'émission lumineuse est susceptible d'être modifiée de manière détectable par le développement d'un micro-organisme dans ledit milieu réactionnel,
(b) on met en présence ledit milieu réactionnel avec l'échantillon, puis on incube ledit milieu,
(c) après ou lors de l'incubation, on observe l'émission lumineuse dudit milieu,
**caractérisé en ce que** le marqueur est un composé chimique, fluorescent, répondant à la formule suivante, ou sa forme anionique :

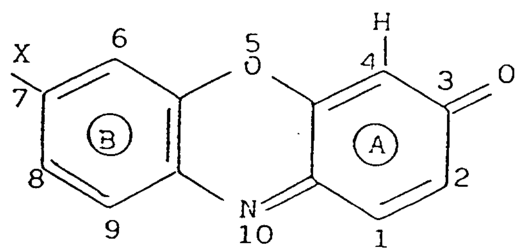

dans laquelle :

- les positions 6, 8, 9 sont occupées chacune par un atome d'hydrogène ou un substituant choisi dans le groupe comprenant le fluor, le chlore, le brome, des substitants alkyle, alkoxy, carboxylique, amide et cyano,
- les positions 1 et 4 sont occupées chacune par un atome d'hydrogène ou un substituant choisi dans le groupe comprenant le fluor, le chlore, le brome, des substituants alkyle, alkoxy, carboxylate, carboxylique, amide et cyano, et la position 2 est occupée par un atome d'hydrogène, ou un substituant choisi dans le groupe comprenant le chlore, le brome et un substituant carboxylique ; ou les positions 1 et 2 appartiennent en commun au noyau A et à un noyau insaturé,

- X est une fonction hydroxyle,

et lors de l'étape (c) on mesure la fluorescence du marqueur, dont la diminution ou l'extinction traduit la présence d'au moins un micro-organisme dans l'échantillon.

**4.** Procédé selon la revendication 3, caractérisé en ce que, pour l'étape (a) le milieu réactionnel est conditionné en ampoule, ou toute autre forme de conditionnement équivalente.

**5.** Procédé selon la revendication 3, caractérisé en ce que, pour l'étape (a) le pH du milieu réactionnel est compris entre 6,0 et 8,0.

**6.** Procédé selon la revendication 3, caractérisé en ce que, pour l'étape (a) le milieu réactionnel comprend une source d'azote.

**7.** Procédé selon la revendication 3, caractérisé en ce que le marqueur est la résorufine ou l'orcirufine.

**8.** Procédé selon la revendication 3, caractérisé en ce que, pour l'étape (a) le marqueur est contenu dans le milieu réactionnel, à raison de 0,01 mg/l à 100 mg/l, et de préférence de 1 à 10 mg/l.

**9.** Procédé selon la revendication 3, caractérisé en ce qu'en effectue l'étape (c) pendant un temps compris entre 1 minute et 7 jours, à une température comprise entre 20 °C et 65 °C.

**10.** Procédé selon la revendication 3, caractérisé en ce que, pour l'étape (a) on utilise une pluralité de milieux réactionnels, différant les uns des autres uniquement par des sources de carbone respectivement différentes.

**11.** Procédé selon la revendication 3, caractérisé en ce que, pour l'étape (a) on utilise une pluralité de milieux réactionnels, différant les uns des autres par l'ajout d'antibiotiques respectivement différents.

**12.** Procédé selon la revendication 3, caractérisé en ce que, pour l'étape (a) on utilise une pluralité de milieux réactionnels, différant les uns des autres par l'ajout d'antifongiques respectivement différents.

**13.** Procédé selon la revendication 3, caractérisé en ce que l'échantillon est prélevé sur du sang, de l'urine ou du liquide céphalo-rachidien.

**14.** Procédé selon la revendication 3, caractérisé en ce que l'échantillon est prélevé sur un produit alimentaire, de l'eau, un produit pharmaceutique ou un produit cosmétique.

**Claims**

**1.** Use of a chemical compound corresponding to the following formula, or its anionic form:

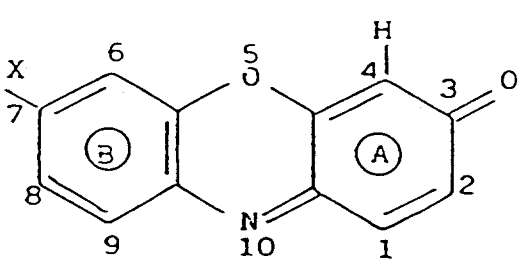

in which:

- positions 6, 8 and 9 are each occupied by a hydrogen atom or a substituent selected from the group comprising fluorine, chlorine, bromine and alkyl, alkoxy, carboxyl, amide and cyano substituents,
- positions 1 and 4 are each occupied by a hydrogen atom or a substituent selected from the group comprising fluorine, chlorine, bromine and alkyl, alkoxy, carboxylate, carboxyl, amide and cyano substituents, and position 2 is occupied by a hydrogen atom, or a substituent selected

from the group comprising chlorine, bromine and a carboxyl substituent; or positions 1 and 2 belong in common to the ring A and to an unsaturated ring, and
- X is a hydroxyl function,

for obtaining a reagent for the detection of microorganisms.

2. Use according to Claim 1, characterized in that the chemical compound is orcirufin or resorufin.

3. Method for the detection of microorganisms in a sample, according to which:
   a) a reaction medium in aqueous phase containing at least one carbon source and a marker, whose luminescent emission is susceptible to being changed in a detectable manner by the growth of a microorganism in said reaction medium, is prepared or obtained;
   b) said reaction medium is contacted with the sample, and this medium is then incubated;
   c) after or during the incubation, the luminescent emission of said medium is observed

   characterized in that the marker is a fluorescent chemical compound corresponding to the following formula, or its anionic form:

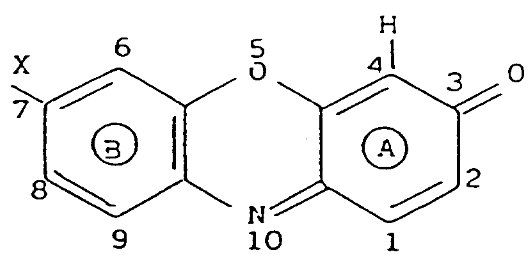

in which:
- positions 6, 8 and 9 are each occupied by a hydrogen atom or a substituent selected from the group comprising fluorine, chlorine, bromine and alkyl, alkoxy, carboxyl, amide and cyano substituents,
- positions 1 and 4 are each occupied by a hydrogen atom or a substituent selected from the group comprising fluorine, chlorine, bromine and alkyl, alkoxy, carboxylate, carboxyl, amide and cyano substituents, and position 2 is occupied by a hydrogen atom, or a substituent selected from the group comprising chlorine, bromine and a carboxyl substituent; or positions 1 and 2 belong in common to the ring A and to an unsaturated ring,
- X is a hydroxyl function,

and in step (c), the fluorescence of the marker is measured, whose diminution or extinction reveals the presence of at least one microorganism in the sample.

4. Mehtod according to Claim 3, characterized in that, for step (a), the reaction medium is enclosed in an ampoule or in any other equivalent form of enclosure.

5. Method according to Claim 3, characterized in that, in step (a), the pH of the reaction medium is between 6.0 and 8.0.

6. Method according to Claim 3, characterized in that, in step (a), the reaction medium comprises a nitrogen source.

7. Method according to Claim 3, characterized in that the marker is resorufin or orcirufin.

8. Method according to Claim 3, characterized in that, in step (a), the marker is contained in the reaction medium in the proportion of 0.01 mg/l to 100 mg/l, and preferably 1 to 10 mg/l.

9. Method according to Claim 3, characterized in that the step (c) is performed for a time of between 1 minute and 7 days, at a temperature of between 20 °C and 65 °C.

**10.** Method according to Claim 3, characterized in that, in step (a), a plurality of reaction media are used, differing from one another only by different respective carbon sources.

**11.** Method according to Claim 3, characterized in that, in step (a), a plurality of reaction media are used, differing from one another by the addition of different respective antibiotics.

**12.** Method according to Claim 3, characterized in that, in step (a), a plurality of reaction media are used, differing from one another by the addition of different respective antifungal agents.

**13.** Method according to Claim 3, characterized in that the sample is withdrawn from blood, urine or cerebrospinal fluid.

**14.** Method according to Claim 3, characterized in that the sample is withdrawn from a foodstuffs product, water, a pharmaceutical product or a cosmetic product.

**Patentansprüche**

**1.** Verwendung einer chemischen Verbindung der folgenden Formel oder deren anionische Form

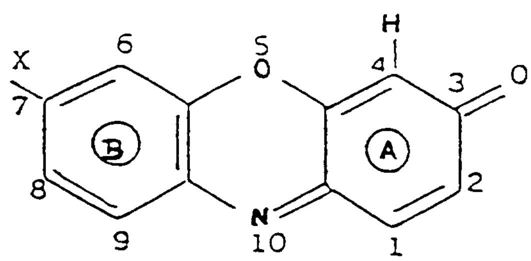

um ein Nachweisreagenz für Mikroorganismen zu erhalten, bei der

- die Stellungen 6, 8, 9 jeweils durch ein Wasserstoffatom oder einen Substituenten, ausgewählt aus der Gruppe bestehend aus Fluor, Chlor, Brom, Alkyl-, Alkoxy-, Carboxyl-, Amino- und Cyanosubstituenten besetzt sind,
- die Stellungen 1 und 4 jeweils durch ein Wasserstoffatom oder einen Substituenten, ausgewählt aus der Gruppe bestehend aus Fluor, Chlor, Brom, Alkyl-, Alkoxy-, Carboxylat-, Carboxyl-, Amino- und Cyanosubstituenten besetzt sind, und die Stellung 2 durch ein Wasserstoffatom oder einen Substituenten, ausgewählt aus der Gruppe bestehend aus Chlor, Brom und einem Carboxylsubstituenten besetzt ist; oder die Stellungen 1 und 2 gemeinsam einem Kern A und einem ungesättigten Kern zugehörig sind, und
- X eine Hydroxylfunktion ist.

**2.** Verwendung nach Anspruch 1, dadurch gekennzeichnet, daß die chemische Verbindung Orcirufin oder Resorufin ist.

**3.** Verfahren zum Nachweisen von Mikroorganismen in einer Probe, bei dem

(a) ein Reaktionsmiliern in wässriger Phase bereitgestellt oder erhalten wird, das zumindest eine Kohlenstoffquelle und einen Marker enthält, dessen Lichtemission in nachweisbarer Form durch die Entwicklung eines Mikroorganismus in dem Reaktionsmilieu modifizierbar ist,

(b) dieses Reaktionsmilieu mit der Probe zusammengebracht wird, anschließend das Milieu inkubiert wird,

(c) wobei nach oder während der Inkubation die Lichtemission des Milieus beobachtet wird,

dadurch gekennzeichnet, daß der Marker eine fluoreszierende chemische Verbindung der folgenden Formel oder deren anionische Form ist, nämlich

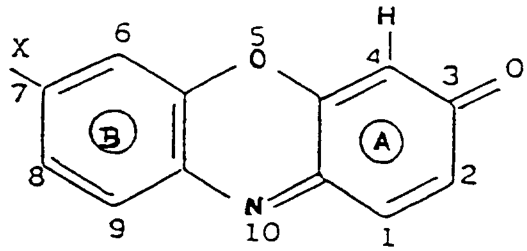

bei der

- die Stellungen 6, 8, 9 jeweils durch ein Wasserstoffatom oder einen Substituenten, ausgewählt aus der Gruppe bestehend aus Fluor, Chlor, Brom, Alkyl-, Alkoxy-, Carboxyl-, Amino- und Cyanosubstituenten besetzt sind,
- die Stellungen 1 und 4 jeweils durch ein Wasserstoffatom oder einen Substituenten, ausgewählt aus der Gruppe bestehend aus Fluor, Chlor, Brom, Alkyl-, Alkoxy-, Carboxylat-, Carboxyl-, Amino- und Cyanosubstituenten besetzt sind, und die Stellung 2 durch ein Wasserstoffatom oder einen Substituenten, ausgewählt aus der Gruppe bestehend aus Chlor, Brom und einem Carboxylsubstituenten besetzt ist; oder die Stellungen 1 und 2 gemeinsam einem Kern A und einem ungesättigten Kern zugehörig sind
- X eine Hydroxylfunktion ist, und wobei im Schritt (c) die Fluoreszenz der Markers gemessen wird, deren Abnahme oder Extinktion die Gegenwart von zumindest einem Mikroorganismus in der Probe wiedergibt.

**4.** Verfahren nach Anspruch 3, dadurch gekennzeichnet, daß in Schritt (a) das Reaktionsmilieu in einer Ampulle oder in jeglicher anderer äquivalenten Konditionierform konditioniert wird.

**5.** Verfahren nach Anspruch 3, dadurch gekennzeichnet, daß in Schritt (a) der pH des Reaktionsmilieus zwischen 6,0 und 8,0 liegt.

**6.** Verfahren nach Anspruch 3, dadurch gekennzeichnet, daß in Schritt (a) das Reaktionsmilieu eine Stickstoffquelle enthält.

**7.** Verfahren nach Anspruch 3, dadurch gekennzeichnet, daß der Marker Orcirufin oder Resorufin ist.

**8.** Verfahren nach Anspruch 3, dadurch gekennzeichnet, daß in Schritt (a) der Marker mit 0,01 mg/l bis 100 mg/l, vorzugsweise mit 1 bis 10 mg/l enthalten ist.

**9.** Verfahren nach Anspruch 3, dadurch gekennzeichnet, daß der Schritt (c) während eines Zeitraumes zwischen 1 Minute und 7 Tagen bei einer Temperatur zwischen 20° und 65°C durchgeführt wird.

**10.** Verfahren nach Anspruch 3, dadurch gekennzeichnet, daß in Schritt (a) eine Vielzahl an Reaktionsmilieus verwendet wird, die sich lediglich durch unterschiedliche Kohlenstoffquellen unterscheiden.

**11.** Verfahren nach Anspruch 3, dadurch gekennzeichnet, daß in Schritt (a) eine Vielzahl an Reaktionsmilieus verwendet wird, die sich durch unterschiedliche antibiotische Zusätze unterscheiden.

**12.** Verfahren nach Anspruch 3, dadurch gekennzeichnet, daß in Schritt (a) eine Vielzahl an Reaktionsmilieus verwendet wird, die sich durch unterschiedliche antifungide Zusätze unterscheiden.

**13.** Verfahren nach Anspruch 3, dadurch gekennzeichnet, daß die Probe aus Blut, Urin oder Gehirn-Rückenmarksflüssigkeit hervorgeht.

**14.** Verfahren nach Anspruch 3, dadurch gekennzeichnet, daß die Probe aus einem Nahrungsmittel, aus Wasser, einem pharmazeutischen oder einem kosmetischen Produkt hervorgeht.